# EUROPÄISCHE PATENTSCHRIFT

(11) **EP 2 300 073 B1**
(45) Veröffentlichungstag und Bekanntmachung des Hinweises auf die Patenterteilung: **29.05.2013**
(21) Anmeldenummer: 09777340.2
(22) Anmeldetag: 21.07.2009
(51) Int. Cl.: A61M 1/36

(54) **VERFAHREN ZUM BETRIEB EINER DRUCKMESSEINRICHTUNG, VORRICHTUNG MIT WENIGSTENS EINER DRUCKMESSEINRICHTUNG, GERÄT MIT DERARTIGEN VORRICHTUNG SOWIE VERWENDUNG EINER MESSKAMMER**
METHOD FOR OPERATING A PRESSURE MEASURING UNIT, APPARATUS HAVING AT LEAST ONE PRESSURE MEASURING UNIT, DEVICE HAVING SUCH AN APPARATUS, AND USE OF A MEASURING CHAMBER
PROCÉDÉ DE FONCTIONNEMENT D'UN APPAREIL DE MESURE DE PRESSION, DISPOSITIF AYANT AU MOINS UN APPAREIL DE MESURE DE PRESSION, APPAREIL DOTÉ DE CE DISPOSITIF ET UTILISATION D'UNE CHAMBRE DE MESURE

(30) Priorität: 22.07.2008 DE 102008034154
(43) Veröffentlichungstag der Anmeldung: 30.03.2011
(73) Patentinhaber: Fresenius Medical Care Deutschland GmbH, 61352 Bad Homburg (DE)
(72) Erfinder: BEDEN, Josef, 55252 Mainz-Kastel (DE); BADO, Itka, 60385 Frankfurt (DE); SPALT, Patrick, 61449 Steinbach (DE)
(74) Vertreter: Herrmann, Uwe
(86) Internationale Anmeldenummer: PCT/EP2009/005292
(87) Internationale Veröffentlichungsnummer: WO 2010/009867

(56) Entgegenhaltungen:
- EP-A1- 1 213 033
- WO-A2-02/03854
- WO-A2-99/13926
- DE-A1-102006 048 785
- US-A- 5 614 677
- US-B1- 6 484 383

## Beschreibung

Die vorliegende Erfindung betrifft ein Verfahren zum Betrieb einer Druckmesseinrichtung zur Messung des Druckes in einem fluiddurchströmten System, insbesondere in einem extrakorporalen Kreislauf eines medizinischen Gerätes, wobei die Druckmesseinrichtung eine durch eine flexible Membran begrenzte Messkammer, die im Betrieb des Systems von Fluid durchströmt wird oder mit Fluid gefüllt ist, sowie einen Drucksensor aufweist, der zum Zwecke der Druckmessung mit der Membran in Verbindung steht.

Ein derartiges Verfahren ist beispielsweise aus der US 5,614,677 bekannt. Die aus dieser Druckschrift bekannte Druckmesseinrichtung dient zur Druckmessung in einem extrakorporalen Blutkreislauf. Dabei wird die Messkammer von Blut durchflossen. Über eine flexible Membran steht die Messkammer mit einem Drucksensor in Kontakt.

Um möglichst zu verhindern, dass es bei einem zu großen Unterdruck zu einer Ablösung der Membran kommt und sich dadurch verfälschte oder überhaupt keine Druckwerte mehr erhalten lassen, wird in der US 5,614,677 vorgeschlagen, die genannte Membran der Messkammer und den Drucksensor möglichst luftfrei aneinanderzupressen. Durch die entsprechende Haftwirkung kann zumindest in einem gewissen Unterdruckbereich eine zuverlässige Messung durchgeführt werden.

Ein Verfahren zum Ankoppeln einer Pumpkammer an die Arbeitsfluidkammer einer Membranpumpe ist aus der US 6,484,383 B1 bekannt.

Aus der EP 1 213 033 A1 ist bereits ein Verfahren zum Betrieb einer Druckmesseinrichtung zur Messung des Druckes in einem fluiddurchströmten System bekannt, wobei die Druckmesseinrichtung eine durch eine flexible Membran begrenzte Messkammer, die im Betrieb des Systems von Fluid durchströmt wird oder mit Fluid gefüllt ist, sowie einen Drucksensor aufweist, der zum Zwecke der Druckmessung mit der Membran in Verbindung steht.

In der sogenannten Akutdialyse erfolgt die Behandlung der Patienten anders als bei der Behandlung chronisch kranker Dialysepatienten, die sich alle zwei bis drei Tage ca. vier bis fünf Stunden einer Hämodialysebehandlung unterziehen müssen, nahezu kontinuierlich. Dabei kann es vorkommen, dass je nach Zustand des Patienten beispielsweise auf einer Intensivstation die Behandlung mehrere Tage ununterbrochen andauern kann. In einem solchen Fall muss dementsprechend über einen vergleichsweise langen Zeitraum sichergestellt werden, dass die Druckmesseinrichtung einwandfrei und zuverlässig arbeitet.

Der vorliegenden Erfindung liegt daher die Aufgabe zugrunde, ein Verfahren der eingangs genannten Art dahingehend weiterzubilden, dass die Druckerfassung auch über einen langen Zeitraum zuverlässig erfolgt.

Diese Aufgabe wird durch ein Verfahren mit den Merkmalen des Anspruchs 1 gelöst.

Danach ist vorgesehen, dass das Verfahren die folgenden, kein Zutun des Personals erfordernden Schritte umfaßt:
a. Erhöhung des Druckes in der Messkammer auf einen Druckwert, bei dem die Membran im nicht mit dem Drucksensor in Verbindung stehenden Zustand relativ zur Messkammer zumindest geringfügig nach außen gewölbt ist, oder Ermittlung eines solchen Druckwertes,
b. Trennen der Membran der Messkammer von dem Drucksensor,
c. Zusammenführen der Membran der Messkammer und des Drucksensors derart, dass die Membran der Messkammer an dem Drucksensor anliegt und
d. Wiederholen der Schritte a. bis c. in vorbestimmten Zeitintervallen und/oder bei Auftreten eines Fehlerfalls.

Erfindungsgemäß ist somit vorgesehen, dass in periodischen Zeitabständen oder bei Auftreten eines Fehlerfalls, d. h. im Bedarfsfall eine mechanische Neuankopplung der Messkammer bzw. deren Membran an dem Drucksensor erfolgt.

Um eine erneute Ankopplung zuverlässig durchführen zu können, das heißt die Membran der Messkammer korrekt an den Drucksensor anlegen zu können, ist erfindungsgemäß vorgesehen, einen derartigen Überdruck in der Messkammer zu erzeugen, dass deren Membran zumindest geringfügig nach außen, das heißt konvex ausgewölbt ist. In diesem Zustand kann eine zuverlässige Kopplung der Membran mit dem Drucksensor erfolgen. Andernfalls besteht die Gefahr, dass die Membran durch einen Unterdruck bei geöffneter Druckmesseinrichtung nach innen gezogen, das heißt konkav verformt wird, was zur Folge hat, dass sich nach der Neuankopplung die Druckmessung unter Umständen verschlechtert bzw. ausfällt.

Durch die Automatisierung des Ablaufs der Verfahrensschritte gemäß Anspruch 1 und die damit verbundene zyklische Wiederholung bzw. das Triggern der Verfahrensschritte a. bis c. bei erkennbaren Problemen mit der Druckmessung, z. B. durch einen Ankoppeltest oder durch Analyse der Druckpulsvarianz am Sensor oder auch anderen Fehlerfällen ist die korrekte Ankopplung über den gesamten Behandlungszeitraum gewährleistet. Ein Versagen der Druckmessung oder eine sonst über einen längeren Zeitraum zu beobachtende Drift zu höheren Drücken hin, kann erfindungsgemäß verhindert werden. Mittels des erfindungsgemäßen Verfahrens lässt sich jederzeit ein hinreichender Überdruck in der Messkammer erzeugen, wodurch eine gute Verbindung mit dem Drucksensor erreicht wird. Abgesehen davon, ist das Verfahren sicher zu jedem Zeit- und Druckpunkt sowie schnell durchführbar.

In bevorzugter Ausgestaltung des Verfahrens ist vorgesehen, dass die Erhöhung des Druckes in der Messkammer mittels einer im System befindlichen Pumpe, insbesondere mittels einer im extrakorporalen Kreislauf befindlichen Blutpumpe, durchgeführt wird. Dabei kann vorgesehen sein, dass die Pumpe zum Zwecke der Erhöhung des Druckes in der Messkammer im Rückwärtslauf betrieben wird, das heißt in einer Förderrichtung, die der Förderrichtung im normalen Betrieb entgegengesetzt ist. Dies ist jedoch kein zwingendes Merkmal. Befindet sich die Druckmesseinrichtung in dem Bereich, der im normalen Betrieb der Pumpe auf der Pumpendruckseite liegt, ist eine Umkehr der Förderrichtung der Pumpe nicht erforderlich.

In weiterer Ausgestaltung der Erfindung ist vorgesehen, dass vor oder während des Betriebes der Pumpe eine Klemme geschlossen wird, um die Durchströmung des Systems zu verhindern oder der Durchströmung einen erhöhten Widerstand entgegenzusetzen, wobei die Klemme derart angeordnet ist, dass sich die Messkammer zwischen Pumpe und Klemme befindet.

In weiterer Ausgestaltung der Erfindung ist vorgesehen, dass Schritt a. vor Schritt b. oder auch Schritt b. vor Schritt a. ausgeführt wird. Vorzugsweise ist vorgesehen, dass die Erhöhung des Druckes der Messkammer auf einen Druckwert bzw. die Feststellung eines solchen Druckwertes erfolgt, bevor die Membran der Messkammer von dem Drucksensor entfernt wird. Grundsätzlich ist jedoch ebenfalls denkbar, zunächst die Membran der Messkammer von dem Drucksensor zu entfernen und sodann die Erhöhung oder Feststellung des Druckes in der Messkammer vorzunehmen. In diesem Fall könnte vorgesehen sein, dass ein vorgegebenes Volumen des Fluids in die Meßkammer gefördert wird.

Gemäß einer weiteren Ausgestaltung der Erfindung ist vorgesehen, dass die Erhöhung des Druckes vorgenommen wird, bis ein vorgegebener Druckwert oder Druckwertbereich erreicht ist. Ist dies nicht der Fall, kann vorgesehen sein, dass die Pumpe weiter fördert, bis der Druckwert erreicht ist. Ist der Druckwert erreicht, kann weiterhin vorgesehen sein, dass die Pumpe angehalten wird.

In weiterer Ausgestaltung der Erfindung ist vorgesehen, dass die Pumpe zum Zwecke der Druckerhöhung ausgehend von einer definierten Stellung des Förderorgans der Pumpe erfolgt. Insbesondere im Falle der Verwendung einer peristaltischen Pumpe ist es sinnvoll, das Anfahren eines Überdrucks vorteilhaft aus einer definierten Lage des Rotors heraus durchzuführen, damit der eingeschlossene Unterdruck im Pumpsegment zwischen den Rollen nicht die gewünschte Überdruckerzeugung verhindert oder behindert.

In weiterer Ausgestaltung der Erfindung ist vorgesehen, dass die Pumpe zum Zwecke der Druckerhöhung solange fördert, bis ein vorgegebenes Fördervolumen oder eine vorgegebene Stellung des Förderorgans der Pumpe erreicht ist. Denkbar ist es beispielsweise, im Falle der genannten peristaltischen Pumpe den Drehwinkel des Rotors zu prüfen und die Förderung zu stoppen, sobald ein bestimmter Drehwinkel erreicht ist.

In weiterer Ausgestaltung der Erfindung ist vorgesehen, dass vor Schritt b., das heißt vor der Trennung der Membran der Messkammer von dem Drucksensor und nach Schritt c., das heißt nach der Neukonnektierung jeweils der Druck in der Messkammer gemessen wird und dass eine Fehlermeldung ausgegeben wird, wenn die beiden Druckwerte nicht übereinstimmen oder nicht in einem vorgegebenen Bereich liegen. Denkbar ist es, dass die genannten Druckmessungen unmittelbar vor dem Abkoppeln und nach der Neuankopplung durchgeführt werden.

In weiterer Ausgestaltung der Erfindung ist vorgesehen, dass die Klemme geöffnet wird, wenn die Membran der Messkammer und der Drucksensor gemäß Schritt c. zusammengeführt sind.

Denkbar ist es, vor dem Schließen der Klemme, gegen die die Pumpe fördert, und nach dem genannten Öffnen der Klemme jeweils den Druck in der Messkammer zu messen und eine Fehlermeldung auszugeben, wenn die beiden Druckwerte nicht übereinstimmen oder nicht in einem vorgegebenen Bereich liegen.

In weiterer Ausgestaltung der Erfindung ist vorgesehen, dass der Drucksensor nach dem Trennen der Membran der Messkammer von dem Drucksensor gemäß Schritt b. und vor dem Zusammenführen der Membran der Messkammer und des Drucksensors gemäß Schritt c. einer Funktionsüberprüfung unterzogen wird. Durch das zyklische Auffahren der Druckmesseinrichtung kann somit gegebenenfalls ein erforderlicher Test des Drucksensors durchgeführt werden. Der Drucksensor kann im nicht angekoppelten Zustand kurze Zeit gegen Umgebung oder gegen einen Referenzdruck geschaltet werden und auf seinen Nullpunkt bzw. auf einen Referenzpunkt und damit auf seine Funktion geprüft werden.

Die vorliegende Erfindung betrifft des weiteren eine Vorrichtung mit den Merkmalen des Anspruchs 14.

Danach sind erste Mittel zur Erhöhung des Druckes in der Messkammer auf einen Druckwert, bei dem die Membran im nicht mit dem Drucksensor in Verbindung stehenden Zustand relativ zur Messkammer zumindest geringfügig nach außen gewölbt ist, oder zur Ermittlung eines solchen Druckwertes vorgesehen. Die Vorrichtung weist des weiteren zweite Mittel zum Trennen der Membran der Messkammer von dem Drucksensor sowie dritte Mittel zum Zusammenführen der Membran der Messkammer und des Drucksensors derart auf, dass die Membran der Messkammer an dem Drucksensor anliegt. Die Vorrichtung weist des weiteren eine Steuereinheit auf, die derart ausgeführt ist, dass sie die ersten, zweiten und dritten Mittel entsprechend ihrer vorgesehenen Betriebsweise in vorbestimmten Zeitintervallen und/oder bei Auftreten eines Fehlerfalls ohne Intervention des Personals ansteuert.

Die Vorrichtung weist vorzugsweise Mittel zur Durchführung der Verfahrensschritte gemäß einem der Ansprüche 1 bis 13 auf.

Bevorzugte Ausgestaltungen der Vorrichtung sind Gegenstand der Ansprüche 15 bis 27.

In einer bevorzugten Ausgestaltung der Erfindung ist die Messkammer als Disposable ausgeführt oder Bestandteil eines Disposables. Denkbar ist es somit, die Messkammer nach der Behandlung eines Patienten auszutauschen. Vorzugsweise ist weiter vorgesehen, dass der Drucksensor als wiederverwendbares Teil oder als Bestandteil einer wiederverwendbaren Einheit ausgeführt ist, das heißt jedenfalls nicht nach jeder Behandlung, sondern nur im Bedarfsfalle ausgetauscht wird.

Die vorliegende Erfindung betrifft des weiteren ein Gerät, insbesondere ein medizinisches Gerät mit den Merkmalen des Anspruchs 28. Bei dem Gerät kann es sich beispielsweise um ein Dialysegerät, um ein Gerät zur Leberunterstützung oder um ein Gerät zur Apheresebehandlung handeln.

Die vorliegende Erfindung betrifft des weiteren die Verwendung einer durch eine flexible Membran begrenzten Messkammer die vorzugsweise einen Fluidzufluss und einen Fluidabfluss aufweist, als Bestandteil einer Druckmesseinrichtung in einem Verfahren gemäß einem der Ansprüche 1 bis 13 und/oder in einer Vorrichtung gemäß einem der Ansprüche 14 bis 27 und/oder in einem Gerät gemäß einem der Ansprüche 28 oder 29.

Wie oben ausgeführt, kann vorgesehen sein, dass die Messkammer als Disposable ausgeführt ist und nach Abschluß vorzugsweise einer Behandlung verworfen wird.

Weitere Einzelheiten und Vorteile der Erfindung werden anhand eines in der Zeichnung dargestellten Ausführungsbeispiels näher erläutert. Dabei zeigen:
- Figur 1:: eine schematische Darstellung des Ablaufes der Neuankopplung der Membran der Messkammer an den Drucksensor gemäß der vorliegenden Erfindung und
- Figur 2:: einen Flußplan zur Durchführung der Neuankopplung der Membran der Messkammer an den Drucksensor gemäß der vorliegenden Erfindung, wobei alle angegebenen Zahlenwerte nur Beispielcharakter haben.

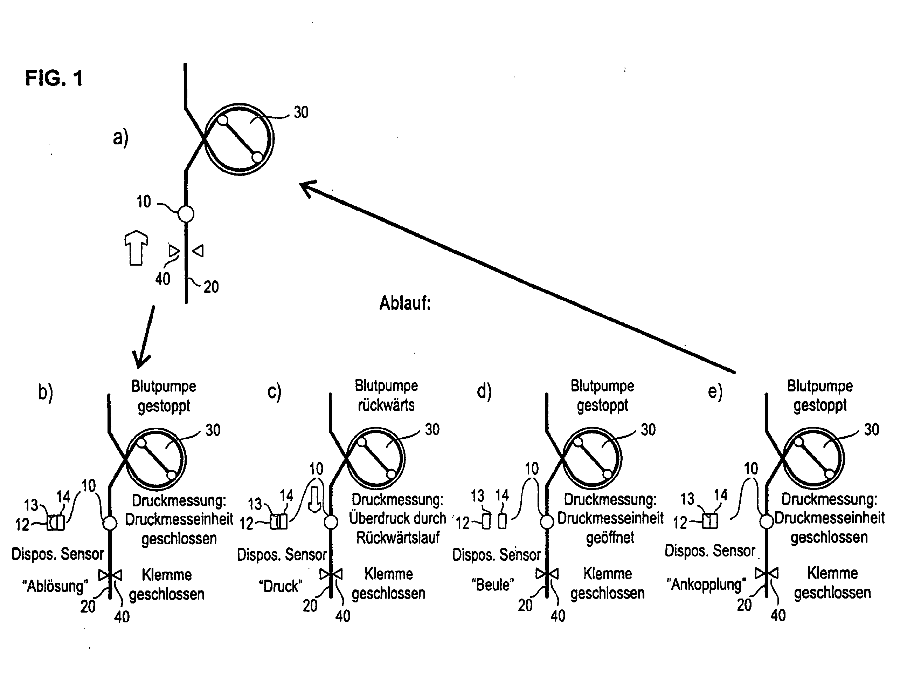

Figur 1 a zeigt mit dem Bezugszeichen 10 eine Druckmesseinrichtung zur Messung des Druckes in einem extrakorporalen Kreislauf 20 eines Dialysegerätes. Mit dem Bezugszeichen 30 ist eine im extrakorporalen Kreislauf 20 befindliche Blutpumpe gekennzeichnet. Das Bezugszeichen 40 kennzeichnet eine Klemme, mittels derer die Schlauchleitung des extrakorporalen Kreislaufes 20 absperrbar ist.

Wie dies aus Figur 1a hervorgeht, befindet sich die Druckmesseinrichtung 10 zwischen der genannten Klemme 40 und der Blutpumpe 30 im extrakorporalen Kreislauf 20 eines Dialysegerätes. Im normalen Betrieb fördert die Blutpumpe 30 in Pfeilrichtung gemäß Figur 1a, was in anderen Worten bedeutet, dass sich die Druckmesseinrichtung 10 auf der Saugseite der Blutpumpe 30 befindet. Im normalen Betrieb können somit in der Messkammer 12, die in den Figuren 1b bis 1e angedeutet ist, vergleichsweise geringe Drücke auftreten, bei denen die Gefahr der Abkopplung der Membran 13 von dem ebenfalls in den Figuren 1b bis 1 e dargestellten Drucksensor 14 vergrößert ist.

Gemäß dem hier dargestellten Ausführungsbeispiel ist vorgesehen, dass in vorbestimmten Zeitintervallen, das heißt periodisch oder im Fehlerfall eine Abkopplung, das heißt eine Trennung der Membran 13 der Messkammer 12 von dem Drucksensor 14 erfolgt und anschließend eine Neuankopplung der Membran 13 der Messkammer 12 an den Drucksensor 14. Durch diese Neuankopplung wird ein guter mechanischer Kontakt weiter sichergestellt. Die Behandlung kann dann normal weitergeführt werden, wohingegen sie während des Ab- und Ankoppelvorgangs vorzugsweise unterbrochen ist. Sofern eine Neuankopplung vorgesehen ist, etwa weil eine vorbestimmte Zeitspanne abgelaufen ist oder weil ein Fehlerfall, beispielsweise die ungewollte Ablösung der Membran 13 der Messkammer 12 von dem Drucksensor 14 festgestellt wird, wird ausgehend von dem Zustand gemäß Figur 1 a die Blutpumpe 30 gestoppt und die Klemme 40 geschlossen, wie dies in Figur 1b dargestellt ist. Sodann wird gemäß Figur 1c die Blutpumpe rückwärts, das heißt entgegen der normalen Förderrichtung betrieben, wodurch sich eine Durchströmung in der Pfeilrichtung gemäß Figur 1c ergibt. Aufgrund der geschlossenen Klemme 40 kommt es somit zu einem Überdruck bzw. Druckaufbau in der Messkammer 12 der Druckmesseinrichtung 10. Dabei kann vorgesehen sein, dass der Druckaufbau aus Sicherheitsgründen in der Höhe begrenzt ist. Alternativ oder zusätzlich kann vorgesehen sein, den Drehwinkel der als peristaltische Pumpe ausgeführten Blutpumpe 30 zu überwachen und/oder das geförderte Volumen zu erfassen. Vorzugsweise wird der Rückwärtslauf der Blutpumpe 30 durchgeführt, bis ein ausreichender Druck aufgebaut ist.

Um zu verhindern, dass im Falle einer peristaltischen Pumpe der eingeschlossene Unterdruck im Pumpsegment zwischen den Rollen die gewünschte Überdruckerzeugung behindert oder verhindert, ist gemäß dem hier dargestellten Ausführungsbeispiel vorgesehen, dass das Anfahren der Pumpe 30 bzw. das Beginnen des Aufbaus des Überdrucks in der Messkammer 12 aus einer definierten Lage des Rotors der Pumpe 30 heraus erfolgt.

Ist der gewünschte Druckwert in der Messkammer 12 erreicht, wird gemäß Schritt 1d die Druckmesseinheit 10 geöffnet, was mit anderen Worten bedeutet, dass die Membran 13 der Messkammer 12 von dem Drucksensor 14 getrennt wird. Aufgrund des in der Messkammer 12 herrschenden Überdruckes wird die Membran 13 der Messkammer nach außen gewölbt, wie dies in Figur 1d angedeutet ist. Es kommt dementsprechend zu einem Ausbeulen der Membran 13 der Messkammer 12.

Sodann wird gemäß Schritt 1e eine Neuankopplung durchgeführt, das heißt die konvex verformte Membran 13 an den Drucksensor 14 angelegt.

Nach Durchführung eines Ankopplungstestes, bei dem festgestellt werden kann, ob die Ankopplung zuverlässig erfolgt ist, kann vorgesehen sein, dass die Klemme 40 wieder geöffnet wird und die Blutpumpe 30 wieder in ihrer normalen Förderrichtung gemäß Figur 1a betrieben wird.

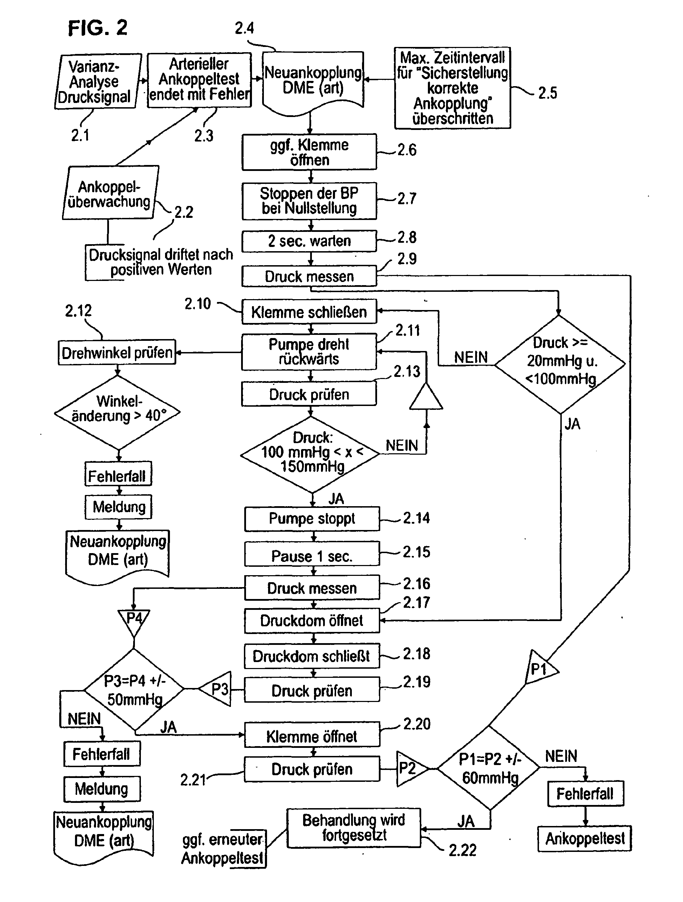

Figur 2 zeigt ein exemplarisches Flußdiagramm, dass die erfindungsgemäße Neuankopplung der Membran 13 der Messkammer 12 an den Drucksensor 14 beschreibt. Gemäß Schritt 2.1 kann eine Varianzanalyse des Drucksignals erfolgen oder gemäß Schritt 2.2 eine Überprüfung des zeitlichen Verlaufs des Drucksignals durchgeführt werden. Enden diese Überprüfungen mit einem Fehler gemäß Schritt 2.3, wird eine Neuankopplung gemäß Schritt 2.4 eingeleitet.

Alternativ oder zusätzlich kann vorgesehen sein, dass gemäß Schritt 2.5 nach Ablauf eines Zeitintervalls, dass abhängig von der Gegebenheit bei typisch ca. ≥ 12 Stunden liegen kann, die Neuankopplung gemäß Schritt 2.4 durchgeführt wird.

Die Neuankopplung beginnt damit, dass gemäß Schritt 2.6 die Klemme, sofern nötig, geöffnet wird und dass anschließend gemäß Schritt 2.7 die Blutpumpe 30 bei einer Vorzugsstellung, die hier als Nullstellung wiedergegeben ist, gestoppt wird. Nach Ablauf einer Wartezeit von beispielsweise 2 Sekunden gemäß Schritt 2.8 wird in Schritt 2.9 der Druck in der Messkammer 12 gemessen. Liegt dieser oberhalb eines Grenzwertes bzw. in einem vorgegebenen Bereich, der in dem hier dargestellten Ausführungsbeispiel zwischen 20 mmHg und 100 mmHg liegt, wird eine Abkopplung der Membran 13 der Messkammer 12 von dem Drucksensor 14 durchgeführt.

Ist dies jedoch nicht der Fall, wird festgestellt, dass eine Druckerhöhung in der Messkammer 12 vorzunehmen ist. In diesem Fall wird die Klemme geschlossen (Schritt 2.10) und die Blutpumpe 30 rückwärts betrieben (Schritt 2.11). In Schritt 2.12 wird der Drehwinkel der Pumpe geprüft. Kommt es zu einer nicht gewünschten Winkeländerung, wird eine Fehlermeldung ausgegeben. Die Neuankopplung kann dann erneut begonnen werden.

Nach Anlaufen der Pumpe 30 wird der Druck geprüft (Schritt 2.13). Wird festgestellt, dass der Druck in einem vorgegebenen Druckwertbereich liegt, wird die Pumpe gestoppt (Schritt 2.14). Ist dies nicht der Fall, wird die Pumpe weiter betrieben.

Nach dem Anhalten der Blutpumpe 30 wird eine vorbestimmte Zeitspanne, hier eine Sekunde, abgewartet (Schritt 2.15) und sodann der Druck gemessen (Schritt 2.16). Dieser Druckwert ist in Figur 2 als P4 dargestellt. In Schritt 2.17 wird der sogenannte Druckdom geöffnet, was in anderen Worten bedeutet, dass die Membran 13 der Messkammer 12 von dem Drucksensor 14 entfernt wird. Anschließend wird eine Neuankopplung durchgeführt ("Druckdom schließt" in Schritt 2.18) und sodann der Druck geprüft (Schritt 2.19). Dieser Druckmesswert ist in Figur 2 mit P3 gekennzeichnet. Stimmen die Druckwerte P3 und P4 nicht innerhalb eines Toleranzbandes überein, wird eine Fehlermeldung ausgegeben und die Neuankopplung erneut begonnen. Ist dies jedoch nicht der Fall, wird die Klemme wieder geöffnet (Schritt 2.20) und sodann der Druck in der Messkammer 12 erneut geprüft (Schritt 2.21). Der dabei ermittelte Druckwert ist in Figur 2 mit P2 gekennzeichnet. Stimmt der vor dem Schließen der Klemme gemessene Druck P1 nicht innerhalb eines Toleranzbandes mit dem Druck P2 überein, wird ein Fehlerfall gemeldet und der sogenannte Ankoppeltest durchgeführt.

Stimmen beide Druckwerte jedoch innerhalb des Toleranzbandes überein, wird die Behandlung fortgesetzt, das heißt die Pumpe in ihrer normalen Förderrichtung in Betrieb gesetzt und die Behandlung fortgeführt (Schritt 2.22).

Durch die vorliegende Erfindung wird sichergestellt, dass über einen langen Zeitraum hin ein Abkoppeln der Membran 13 der Messkammer 12 von dem Drucksensor 14 ausgeschlossen wird bzw. auch bei im extrakorporalen Kreislauf 20 herrschenden Unterdruck korrigiert werden kann. Dadurch werden zuverlässige Druckmessungen sichergestellt.

Die vorliegende Erfindung ist insbesondere dann von Bedeutung, wenn die Druckmessung in einem Bereich erfolgt, der im normalen Betrieb auf der Unterdruckseite, das heißt auf der Saugseite einer Pumpe liegt und in den auch bei gestoppter Flüssigkeitspumpe ein negativer Druck herrschen kann. Die Erfindung ist jedoch darauf nicht beschränkt und umfasst auch Fälle, in denen die Druckmesseinrichtung auf der Druckseite einer Pumpe liegt.

Das erfindungsgemäße Verfahren bzw. die erfindungsgemäße Vorrichtung können beispielsweise in einem multifunktionalen Gerät zur Behandlung akuter Nierenerkrankungen und/oder zum Einsatz in einer Leberunterstützung und/oder zur Anwendung bei verschiedenen therapeutischen Aphereseverfahren eingesetzt werden. Auch andere Anwendungsfälle sind denkbar.

Der Drucksensor 14 kann wie auch die Messkammer 12 mit einer Membran ausgeführt sein, an die im normalen Betrieb die Membran 13 der Messkammer 12 anliegt. Denkbar ist beispielsweise eine Anordnung, wie sie aus der US 5,614,677 bekannt ist.

## Patentansprüche

1. Verfahren zum Betrieb einer Druckmesseinrichtung (10) zur Messung des Druckes in einem fluiddurchströmten System, insbesondere in einem extrakorporalen Kreislauf (20) eines medizinischen Gerätes, wobei die Druckmesseinrichtung (10) eine durch eine flexible Membran (13) begrenzte Messkammer (12), die im Betrieb des Systems von Fluid durchströmt wird oder mit Fluid gefüllt ist, sowie einen Drucksensor (14) aufweist, der zum Zwecke der Druckmessung mit der Membran (13) in Verbindung steht,
**dadurch gekennzeichnet,**
**dass** das Verfahren die folgenden, keine Intervention des Personals erfordernden Schritte umfaßt:
a. Erhöhung des Druckes in der Messkammer (12) auf einen Druckwert, bei dem die Membran (13) im nicht mit dem Drucksensor (14) in Verbindung stehenden Zustand relativ zur Messkammer (12) zumindest geringfügig nach außen gewölbt ist, oder Ermittlung eines solchen Druckwertes,
b. Trennen der Membran (13) der Messkammer (12) von dem Drucksensor (14),
c. Zusammenführen der Membran (13) der Messkammer (12) und des Drucksensors (14) derart, dass die Membran (13) der Messkammer (12) an dem Drucksensor (14) anliegt und
d. Wiederholen der Schritte a. bis c. in vorbestimmten Zeitintervallen und/oder bei Auftreten eines Fehlerfalls.

2. Verfahren nach Anspruch 1, **dadurch gekennzeichnet, dass** die Erhöhung des Druckes in der Messkammer (12) mittels einer im System befindlichen Pumpe (30), insbesondere mittels einer im extrakorporalen Kreislauf (20) befindlichen Blutpumpe (30), durchgeführt wird.

3. Verfahren nach Anspruch 2, **dadurch gekennzeichnet, dass** die Pumpe (30) zum Zwecke der Erhöhung des Druckes in der Messkammer (12) im Rückwärtslauf betrieben wird.

4. Verfahren nach Anspruch 2 oder 3, **dadurch gekennzeichnet, dass** vor oder während des Betriebes der Pumpe (30) eine Klemme (40) geschlossen wird, um die Durchströmung des Systems zu verhindern oder der Durchströmung einen erhöhten Widerstand entgegenzusetzen, wobei die Klemme (40) derart angeordnet ist, dass sich die Messkammer (12) zwischen Pumpe (30) und Klemme (40) befindet.

5. Verfahren nach einem der vorhergehenden Ansprüche, **dadurch gekennzeichnet, dass** Schritt a. vor Schritt b. oder Schritt b. vor Schritt a. ausgeführt wird.

6. Verfahren nach einem der vorhergehenden Ansprüche, **dadurch gekennzeichnet, dass** die Erhöhung des Druckes vorgenommen wird, bis ein vorgegebener Druckwert oder Druckwertbereich erreicht ist.

7. Verfahren nach Anspruch 6 sowie nach einem der Ansprüche 2 bis 5, **dadurch gekennzeichnet, dass** die Pumpe (30) gestoppt wird, wenn der vorgegebene Druckwert oder Druckwertbereich erreicht ist.

8. Verfahren nach einem der Ansprüche 2 bis 7, **dadurch gekennzeichnet, dass** die Pumpe (30) zum Zwecke der Druckerhöhung ausgehend von einer definierten Stellung des Förderorgans der Pumpe (30) in Betrieb gesetzt wird.

9. Verfahren nach einem der Ansprüche 2 bis 8, **dadurch gekennzeichnet, dass** die Pumpe (30) zum Zwecke der Druckerhöhung solange fördert, bis ein vorgegebenes Fördervolumen oder bis eine vorgegebene Stellung des Förderorgans der Pumpe (30) erreicht ist.

10. Verfahren nach einem der vorhergehenden Ansprüche, **dadurch gekennzeichnet, dass** vor Schritt b. und nach Schritt c. der Druck in der Messkammer (12) gemessen wird und dass eine Fehlermeldung ausgegeben wird, wenn die Druckwerte nicht übereinstimmen oder nicht in einem vorgegebenen Bereich liegen.

11. Verfahren nach einem der Ansprüche 4 bis 10, **dadurch gekennzeichnet, dass** die Klemme (40) geöffnet wird, wenn die Membran (13) der Messkammer (12) und der Drucksensor (14) gemäß Schritt c. zusammengeführt sind.

12. Verfahren nach Anspruch 11, **dadurch gekennzeichnet, dass** vor dem Schließen der Klemme (40) und nach dem Öffnen der Klemme (40) der Druck in der Messkammer (12) gemessen wird und dass eine Fehlermeldung ausgegeben wird, wenn die Druckwerte nicht übereinstimmen oder nicht in einem vorgegebenen Bereich liegen.

13. Verfahren nach einem der vorhergehenden Ansprüche, **dadurch gekennzeichnet, dass** der Drucksensor (14) nach dem Trennen der Membran (13) der Messkammer (12) von dem Drucksensor (14) gemäß Schritt b. und vor dem Zusammenführen der Membran (13) der Messkammer (12) und des Drucksensors (14) gemäß Schritt c. einer Funktionsüberprüfung unterzogen wird.

14. Vorrichtung mit wenigstens einer Druckmesseinrichtung (10) zur Messung des Druckes in einem fluiddurchströmten System, insbesondere in einem extrakorporalen Kreislauf (20) eines medizinischen Gerätes, wobei die Druckmesseinrichtung (10) wenigstens eine durch eine flexible Membran (13) begrenzte Messkammer (12), die im Betrieb des System von Fluid durchströmt wird oder mit Fluid gefüllt ist, sowie wenigstens einen Drucksensor (14) aufweist, der zum Zwecke der Druckmessung mit der Membran (13) in Verbindung steht, wobei die Vorrichtung erste Mittel zur Erhöhung des Druckes in der Messkammer (12) auf einen Druckwert, bei dem die Membran (13) im nicht mit dem Drucksensor (14) in Verbindung stehenden Zustand relativ zur Messkammer (12) zumindest geringfügig nach außen gewölbt ist, oder zur Ermittlung eines solchen Druckwertes, zweite Mittel zum Trennen der Membran (13) der Messkammer (12) von dem Drucksensor (14), sowie dritte Mittel zum Zusammenführen der Membran (13) der Messkammer (12) und des Drucksensors (14) aufweist, die derart ausgebildet sind, dass die Membran (13) der Messkammer (12) an dem Drucksensor (14) anliegt,
**dadurch gekennzeichnet,**
**dass** die Vorrichtung eine Steuereinheit aufweist, die derart ausgeführt ist, dass sie die ersten, zweiten und dritten Mittel in vorbestimmten Zeitintervallen und/oder bei Auftreten eines Fehlerfalls ohne Intervention des Personals ansteuert.

15. Vorrichtung nach Anspruch 14, **dadurch gekennzeichnet, dass** die ersten Mittel eine Pumpe (30), insbesondere eine im extrakorporalen Kreislauf (20) befindliche Blutpumpe (30), umfassen.

16. Vorrichtung nach Anspruch 15, **dadurch gekennzeichnet, dass** die Steuereinheit derart ausgeführt ist, dass sie die Pumpe (30) zum Zwecke der Erhöhung des Druckes in der Messkammer (12) im Rückwärtslauf ansteuert.

17. Vorrichtung nach einem der Ansprüche 14 bis 16, **dadurch gekennzeichnet, dass** die ersten Mittel eine Klemme (40) umfassen, um die Durchströmung des Systems zu verhindern oder der Durchströmung des Systems einen erhöhten Widerstand entgegenzusetzen, wobei die Klemme (40) derart angeordnet ist, dass sich die Messkammer (12) zwischen Pumpe (30) und Klemme (40) befindet und wobei die Steuereinheit derart ausgeführt ist, dass sie das teilweise oder vollständige Schließen der Klemme (40) veranlaßt, bevor oder während die Pumpe (30) zum Zwecke der Erhöhung des Druckes in der Messkammer (12) läuft.

18. Vorrichtung nach einem der Ansprüche 14 bis 17, **dadurch gekennzeichnet, dass** die Steuereinheit derart ausgeführt ist, dass sie die ersten und zweiten Mittel derart ansteuert, dass die ersten Mittel vor den zweiten Mitteln oder die zweiten Mittel vor den ersten Mitteln betrieben werden.

19. Vorrichtung nach einem der Ansprüche 14 bis 18, **dadurch gekennzeichnet, dass** die Steuereinheit derart ausgeführt ist, dass sie eine Erhöhung des Druckes in der Messkammer (12) durch die ersten Mittel veranlaßt, bis ein vorgegebener Druckwert oder Druckwertbereich erreicht ist.

20. Vorrichtung nach einem der Ansprüche 15 bis 19, **dadurch gekennzeichnet, dass** die Steuereinheit derart ausgeführt ist, dass sie die Erhöhung des Druckes in der Messkammer (12) durch die Pumpe (30) ausgehend von einer definierten Stellung des Förderorgans der Pumpe (30) veranlaßt.

21. Vorrichtung nach einem der Ansprüche 15 bis 20, **dadurch gekennzeichnet, dass** die Steuereinheit derart ausgeführt ist, dass sie die Erhöhung des Druckes in der Messkammer (12) durch die Pumpe (30) veranlaßt, bis ein vorgegebenes Fördervolumen oder bis eine vorgegebene Stellung des Förderorgans der Pumpe (30) erreicht ist.

22. Vorrichtung nach einem der Ansprüche 14 bis 21, **dadurch gekennzeichnet, dass** die Steuereinheit derart ausgeführt ist, dass sie vor Aktivierung der zweiten Mittel und nach Aktivierung der dritten Mittel jeweils eine Druckmessung in der Messkammer (12) veranlaßt, und dass eine Einrichtung zur Ausgabe einer Fehlermeldung vorgesehen ist, die derart ausgeführt ist, dass sie durch die Steuereinheit aktiviert wird, wenn die beiden Druckwerte nicht übereinstimmen oder nicht in einem vorgegebenen Bereich liegen.

23. Vorrichtung nach einem der Ansprüche 14 bis 22, **dadurch gekennzeichnet, dass** die Steuereinheit derart ausgeführt ist, dass sie das Öffnen der Klemme (40) veranlaßt, nachdem die Membran (13) der Messkammer (12) und der Drucksensor (14) durch die dritten Mittel zusammengeführt sind.

24. Vorrichtung nach einem der Ansprüche 17 bis 23, **dadurch gekennzeichnet, dass** die Steuereinheit derart ausgeführt ist, dass sie vor dem Schließen der Klemme (40) und nach dem Öffnen der Klemme (40) jeweils eine Druckmessung in der Messkammer (12) veranlaßt, und dass eine Einrichtung zur Ausgabe einer Fehlermeldung vorgesehen ist, die derart ausgeführt ist, dass sie durch die Steuereinheit aktiviert wird, wenn die beiden Druckwerte nicht übereinstimmen oder nicht in einem vorgegebenen Bereich liegen.

25. Vorrichtung nach einem der Ansprüche 14 bis 24, **dadurch gekennzeichnet, dass** die Steuereinheit derart ausgeführt ist, dass sie den Drucksensor (14) nach Aktivierung der zweiten Mittel und vor Aktivierung der dritten Mittel einer Funktionsüberprüfung unterzieht.

26. Vorrichtung nach einem der Ansprüche 14 bis 25, **dadurch gekennzeichnet, dass** die Messkammer (12) als Disposable ausgeführt ist oder Bestandteil eines Disposables ist.

27. Vorrichtung nach einem der Ansprüche 14 bis 26, **dadurch gekennzeichnet, dass** der Drucksensor (14) als wieder verwendbares Teil oder als Bestandteil einer wieder verwendbaren Einheit ausgeführt ist.

28. Gerät, insbesondere medizinisches Gerät mit wenigstens einer Vorrichtung gemäß einem der Ansprüche 14 bis 27.

29. Gerät nach Anspruch 28, **dadurch gekennzeichnet, dass** es sich um ein Dialysegerät, um ein Gerät zur Leberunterstützung oder um ein Gerät zur Apheresebehandlung handelt.

30. Verwendung einer durch eine flexible Membran (13) begrenzten Messkammer (12) mit einem Fluidzufluss und einem Fluidabfluss als Bestandteil einer Druckmesseinrichtung (10) in einem Verfahren gemäß einem der Ansprüche 1 bis 13 und/oder in einer Vorrichtung gemäß einem der Ansprüche 14 bis 27 und/oder in einem Gerät gemäß einem der Ansprüche 28 oder 29.

31. Verwendung nach Anspruch 30, **dadurch gekennzeichnet, dass** die Messkammer (12) als Disposable ausgeführt ist und nach Abschluß vorzugsweise einer Behandlung verworfen wird.

## Claims

1. A method for the operation of a pressure measuring device (10) for the measurement of the pressure in a system through which fluid flows, in particular in an extracorporeal circuit (20) of a medical unit, with the pressure measuring device (10) having a measuring chamber (12) which is bounded by a flexible membrane (13), through which fluid flows in the operation of the system or which is filled with fluid and also having a pressure sensor (14) connected to the membrane (13) for the purpose of pressure measurement,
**characterized in that**
the method includes the following steps not requiring any intervention of the staff:
a. increasing the pressure in the measuring chamber (12) to a pressure value at which the membrane (13) is at least slightly outwardly curved relative to the measuring chamber (12) in the state not connected to the pressure sensor (14) or determining such a pressure value;
b. separating the membrane (13) of the measuring chamber (12) from the pressure sensor (14);
c. joining together the membrane (13) of the measuring chamber (12) and of the pressure sensor (14) such that the membrane (13) of the measuring chamber (12) contacts the pressure sensor (14); and
d. repeating the steps a. to c. at predetermined intervals and/or on the occurrence of an error case.

2. A method in accordance with claim 1, **characterized in that** the increase in the pressure in the measuring chamber (12) is carried out by means of a pump (30) located in the system, in particular by means of a blood pump (30) located in the extracorporeal circuit (20).

3. A method in accordance with claim 2, **characterized in that** the pump (30) is operated in reverse operation for the purpose of increasing the pressure in the measuring chamber (12).

4. A method in accordance with either of claims 2 or 3, **characterized in that** a clamp (40) is closed before or after the operation of the pump (30) to prevent the system being flowed through or to set an increased resistance against the throughflow, with the clamp (40) being arranged such that the measuring chamber (12) is located between the pump (30) and the clamp (40).

5. A method in accordance with any one of the preceding claims, **characterized in that** step a. is carried out before step b. or step b. is carried out before step a.

6. A method in accordance with one of the preceding claims, **characterized in that** the increase of the pressure is carried out until a predetermined pressure value or pressure value range has been reached.

7. A method in accordance with claim 6 as well as in accordance with one of the claims 2 to 5, **characterized in that** the pump (30) is stopped when the preset pressure value or pressure value range has been reached.

8. A method in accordance with one of the claims 2 to 7, **characterized in that** the pump (30) is set into operation for the purpose of the pressure increase, starting from a defined position of the conveying member of the pump (30).

9. A method in accordance with one of the claims 2 to 8, **characterized in that** the pump (30) conveys so long for the purpose of the pressure increase until a preset conveying volume has been reached or until a preset position of the conveying member of the pump (30) has been reached.

10. A method in accordance with one of the preceding claims, **characterized in that** the pressure in the measuring chamber (12) is measured before step b. and after step c.; and **in that** an error message is output if the pressure values do not coincide or if they are not in a preset range.

11. A method in accordance with one of the claims 4 to 10, **characterized in that** the clamp (40) is opened when the membrane (13) of the measuring chamber (12) and the pressure sensor (14) are joined together in accordance with step c.

12. A method in accordance with claim 11, **characterized in that** the pressure in the measuring chamber (12) is measured before the closing of the clamp (40) and after the opening of the clamp (40); and **in that** an error message is output if the pressure values do not coincide or if they are not in a preset range.

13. A method in accordance with one of the preceding claims, **characterized in that** the pressure sensor (14) is subjected to a function test after the separation of the membrane (13) of the measuring chamber (12) from the pressure sensor (14) in accordance with step b. and before the joining together of the membrane (13) of the measuring chamber (12) and of the pressure sensor (14) in accordance with step c.

14. An apparatus having at least one pressure measuring device (10) for the measurement of the pressure in a system through which fluid flows, in particular in an extracorporeal circuit (20) of a medical unit, with the pressure measuring device (10) having at least one measuring chamber (12) which is bounded by a flexible membrane (13), through which fluid flows in the operation of the system or which is filled with fluid and also having at least one pressure sensor (14) connected to the membrane (13) for the purpose of pressure measurement, wherein the apparatus has first means for increasing the pressure in the measuring chamber (12) to a pressure value at which the membrane (13) is at least slightly outwardly curved relative to the measuring chamber (12) in the state not connected to the pressure sensor (14), or for determining such a pressure value; second means for separating the membrane (13) of the measuring chamber (12) from the pressure sensor (14); and third means for joining together the membrane (13) of the measuring chamber (12) and of the pressure sensor (14) which are formed such that the membrane (13) of the measuring chamber (12) contacts the pressure sensor (14),
**characterized in that**
the apparatus has as a control unit which is made such that it controls the first, second and third means at predetermined time intervals and/or on the occurrence of an error case without intervention of the staff.

15. An apparatus in accordance with claim 14, **characterized in that** the first means include a pump (30), in particular a blood pump (30) located in the extracorporeal circuit (20).

16. An apparatus in accordance with claim 15, **characterized in that** the control unit is made such that it controls the pump (30) for the purpose of increasing the pressure in the measuring chamber (12) in reverse operation.

17. An apparatus in accordance with one of the claims 14 to 16, **characterized in that** the first means include a clamp (40) to prevent a throughflow of the system or to set an increased resistance against the throughflow of the system, with the clamp (40) being arranged such that the measuring chamber (12) is located between the pump (30) and the clamp (40), and wherein the control unit is made such that it initiates the partial or complete closing of the clamp (40) before or while the pump (30) runs for the purpose of increasing the pressure in the measuring chamber (12).

18. An apparatus in accordance with one of the claims 14 to 17, **characterized in that** the control unit is made such that it controls the first and second means such that the first means are operated before the second means or the second means are operated before the first means.

19. An apparatus in accordance with one of the claims 14 to 18, **characterized in that** the control unit is made such that it initiates an increase in the pressure in the measuring chamber (12) by the first means until a preset pressure value or pressure value region has been reached.

20. An apparatus in accordance with one of the claims 15 to 19, **characterized in that** the control unit is made such that it initiates the increase of the pressure in the measuring chamber (12) by the pump (30), starting from a defined position of the conveying member of the pump (30).

21. An apparatus in accordance with one of the claims 15 to 20, **characterized in that** the control unit is made such that it initiates the increase of the pressure in the measuring chamber (12) by the pump (30) until a preset conveying volume has been reached or until a preset position of the conveying member of the pump (30) has been reached.

22. An apparatus in accordance with one of the claims 14 to 21, **characterized in that** the control unit is made such that it in each case initiates a pressure measurement in the measuring chamber (12) before activation of the second means and after activation of the third means; and **in that** a device is provided for the outputting of an error message which is made such that it is activated by the control unit if the two pressure values do not coincide or are not in a preset range.

23. An apparatus in accordance with one of the claims 14 to 22, **characterized in that** the control unit is made such that it initiates the opening of the clamp (40) after the membrane (13) of the measuring chamber (12) and the pressure sensor (14) have been joined together by the third means.

24. An apparatus in accordance with one of the claims 17 to 23, **characterized in that** the control unit is made such that it in each case initiates a pressure measurement in the measuring chamber (12) before the closing of the clamp (40) and after the opening of the clamp (40); and **in that** a device is provided for the outputting of an error message which is made such that it is activated by the control unit if the two pressure values do not coincide or are not in a preset range.

25. An apparatus in accordance with one of the claims 14 to 24, **characterized in that** the control unit is made such that it subjects the pressure sensor (14) to a function test after the activation of the second means and before the activation of the third means.

26. An apparatus in accordance with one of the claims 14 to 25, **characterized in that** the measuring chamber (12) is made as a disposable product or is a component of a disposable product.

27. An apparatus in accordance with one of the claims 14 to 26, **characterized in that** the pressure sensor (14) is made as a reusable part or as a component of a reusable unit.

28. A unit, in particular a medical unit, comprising at least one apparatus in accordance with one of the claims 14 to 27.

29. A unit in accordance with claim 28, **characterized in that** it is a dialyzer, a unit to support the liver or a unit for apheresis treatment.

30. Use of a measuring chamber (12) bounded by a flexible membrane (13) and having a fluid inflow and a fluid outflow as a component of a pressure measuring device (10) in a method in accordance with one of the claims 1 to 13 and/or in an apparatus in accordance with one of the claims 14 to 27 and/or in a unit in accordance with one of the claims 28 or 29.

31. Use in accordance with claim 30, **characterized in that** the measuring chamber (12) is made as a disposable product and is discarded after the conclusion of preferably one treatment.

## Revendications

1. Procédé de fonctionnement d'un dispositif manométrique (10) destiné à la mesure de la pression dans un système traversé par un fluide, notamment dans un circuit extracorporel (20) d'un appareil médical, le dispositif manométrique (10) présentant une chambre de mesure (12) limitée par une membrane flexible (13), qui est traversée par du fluide ou remplie de fluide pendant le fonctionnement du système, ainsi qu'un capteur de pression (14), qui se trouve en relation avec la membrane (13) en vue de la mesure de la pression,
**caractérisé en ce que**
le procédé comprend les étapes suivantes, ne nécessitant aucune intervention du personnel :
a. Augmentation de la pression dans la chambre de mesure (12) à une valeur de pression, pour laquelle la membrane (13), à l'état ne se trouvant pas en relation avec le capteur de pression (14) par rapport à la chambre de mesure (12) est bombée au moins de manière minime vers l'extérieur, ou calcul d'une telle valeur de pression,
b. Séparation de la membrane (13) de la chambre de mesure (12) par rapport au capteur de pression (14),
c. Réunion de la membrane (13) de la chambre de mesure (12) et du capteur de pression (14), de sorte que la membrane (13) de la chambre de mesure (12) soit collée au capteur de pression (14).
d. Répétition des étapes a. à c. dans des intervalles de temps prédéterminés et/ou en cas d'apparition d'une erreur.

2. Procédé selon la revendication 1, **caractérisé en ce que** l'augmentation de la pression dans la chambre de mesure (12) est réalisée au moyen d'une pompe (30) se trouvant dans le système, notamment d'une pompe à sang (30) se trouvant dans un circuit extracorporel (20).

3. Procédé selon la revendication 2, **caractérisé en ce que** la pompe (30) est actionnée en marche arrière en vue de l'augmentation de la pression dans la chambre de mesure (12).

4. Procédé selon les revendications 2 ou 3, **caractérisé en ce que**, avant ou pendant le fonctionnement de la pompe (30), une pince (40) est fermée afin d'empêcher la traversée du système ou d'opposer une résistance accrue à la traversée, la pince (40) étant disposée de sorte que la chambre de mesure (12) se trouve entre la pompe (30) et la pince (40).

5. Procédé selon une quelconque des revendications précédentes, **caractérisé en ce que** l'étape a. est réalisée avant l'étape b ou l'étape b. avant l'étape a..

6. Procédé selon une quelconque des revendications précédentes, **caractérisé en ce que** l'augmentation de la pression est effectuée jusqu'à ce qu'une valeur de pression ou une zone de valeur de pression prescrites soient atteintes.

7. Procédé selon la revendication 6 ainsi que selon une quelconque des revendications 2 à 5, **caractérisé en ce que** la pompe (30) est arrêtée lorsque la valeur de pression ou la zone de valeur de pression prescrites sont atteintes.

8. Procédé selon une quelconque des revendications 2 à 7, **caractérisé en ce que** la pompe (30) est mise en service en vue de l'augmentation de la pression en partant d'une position définie de l'organe de transport de la pompe (30).

9. Procédé selon une quelconque des revendications 2 à 8, **caractérisé en ce que** la pompe (30) transporte en vue de l'augmentation de la pression, jusqu'à ce qu'un volume de transport prescrit ou jusqu'à ce qu'une position prescrite de l'organe de transport de la pompe (30) soient atteints.

10. Procédé selon une quelconque des revendications précédentes, **caractérisé en ce que**, avant l'étape b. et après l'étape c., la pression est mesurée dans la chambre de mesure (12) et **en ce qu'**un message d'erreur est émis lorsque les valeurs de pression ne coïncident pas ou ne se trouvent pas dans une zone prescrite.

11. Procédé selon une quelconque des revendications 4 à 10, **caractérisé en ce que**, la pince (40) est ouverte lorsque la membrane (13) de la chambre de mesure (12) et le capteur de pression (14) sont réunies selon l'étape c..

12. Procédé selon la revendication 11, **caractérisé en ce que**, avant la fermeture de la pince (40) et après l'ouverture de la pince (40), la pression est mesurée dans la chambre de mesure (12) et **en ce qu'**un message d'erreur est émis lorsque les valeurs de pression ne coïncident pas ou ne se trouvent pas dans une zone prescrite.

13. Procédé selon une quelconque des revendications précédentes, **caractérisé en ce que** le capteur de pression (14) est soumis à un contrôle de fonctionnement après la séparation de la membrane (13) de la chambre de mesure (12) par rapport au capteur de pression (14) selon l'étape b., et avant la réunion de la membrane (13) de la chambre de mesure (12) et du capteur de pression (14) selon l'étape c..

14. Dispositif avec au moins un dispositif manométrique (10) destiné à la mesure de la pression dans un système traversé par un fluide, notamment dans un circuit extracorporel (20) d'un appareil médical, le dispositif manométrique (10) présentant au moins une chambre de mesure (12) limitée par une membrane flexible (13), qui est traversée par du fluide ou remplie de fluide pendant le fonctionnement du système, ainsi qu'au moins un capteur de pression (14), qui se trouve en relation avec la membrane (13) en vue de la mesure de la pression, le dispositif comprenant des premiers moyens d'augmentation de la pression dans la chambre de mesure (12) à une valeur de pression, pour laquelle la membrane (13), à l'état ne se trouvant pas en relation avec le capteur de pression (14) par rapport à la chambre de mesure (12) est bombée au moins de manière minime vers l'extérieur, ou en vue du calcul d'une telle valeur de pression, des deuxièmes moyens pour séparer la membrane (13) de la chambre de mesure (12) par rapport au capteur de pression (14), ainsi que des troisièmes moyens pour réunir la membrane (13) de la chambre de mesure (12) et le capteur de pression (14), qui sont constitués de sorte que la membrane (13) de la chambre de mesure (12) soit collée au capteur de pression (14),
**caractérisé en ce que**
le dispositif présente une unité de commande qui est réalisée de sorte qu'elle amorce sans intervention du personnel les premiers, les seconds et les troisièmes moyens dans des intervalles de temps prédéterminés et/ou en cas d'apparition d'une erreur.

15. Dispositif selon la revendication 14, **caractérisé en ce que** les premiers moyens comprennent une pompe (30), notamment une pompe à sang (30) se trouvant dans un circuit extracorporel (20).

16. Dispositif selon la revendication 15, **caractérisé en ce que** l'unité de commande est réalisée de sorte qu'elle amorce la pompe (30) en marche arrière en vue de l'augmentation de la pression dans la chambre de mesure (12).

17. Dispositif selon une quelconque des revendications 14 à 16, **caractérisé en ce que** les premiers moyens comprennent une pince (40) afin d'empêcher la traversée du système ou d'opposer une résistance accrue à la traversée du système, la pince (40) étant disposée de sorte que la chambre de mesure (12) se trouve entre la pompe (30) et la pince (40) et l'unité de commande étant réalisée de sorte qu'elle déclenche la fermeture partielle ou totale de la pince (40), avant ou pendant que la pompe (30) fonctionne en vue de l'augmentation de la pression dans la chambre de mesure (12).

18. Dispositif selon une quelconque des revendications 14 à 17, **caractérisé en ce que** l'unité de commande est réalisée de sorte qu'elle amorce les premiers et les seconds moyens de sorte que les premiers moyens sont actionnés avant les seconds moyens ou les seconds moyens avant les premiers moyens.

19. Dispositif selon une quelconque des revendications 14 à 18, **caractérisé en ce que** l'unité de commande est réalisée de sorte qu'elle déclenche une augmentation de la pression dans la chambre de mesure (12) par les premiers moyens jusqu'à ce qu'une valeur de pression ou une zone de valeur de pression prescrites soient atteintes.

20. Dispositif selon une quelconque des revendications 15 à 19, **caractérisé en ce que** l'unité de commande est réalisée de sorte qu'elle déclenche l'augmentation de la pression dans la chambre de mesure (12) par la pompe (30) en partant d'une position définie de l'organe de transport de la pompe (30).

21. Dispositif selon une quelconque des revendications 15 à 20, **caractérisé en ce que** l'unité de commande est réalisée de sorte qu'elle déclenche l'augmentation de la pression dans la chambre de mesure (12) par la pompe (30), jusqu'à ce qu'un volume de transport prescrit ou jusqu'à ce qu'une position prescrite de l'organe de transport de la pompe (30) soient atteints.

22. Dispositif selon une quelconque des revendications 14 à 21, **caractérisé en ce que** l'unité de commande est réalisée de sorte que, avant l'activation des seconds moyens et après l'activation des troisièmes moyens, elle déclenche respectivement une mesure de pression dans la chambre de mesure (12) et **en ce qu'**un dispositif est prévu pour l'émission d'un message d'erreur, qui est réalisé de sorte qu'il est activé par l'unité de commande lorsque les deux valeurs de pression ne coïncident pas ou ne se trouvent pas dans une zone prescrite.

23. Dispositif selon une quelconque des revendications 14 à 22, **caractérisé en ce que** l'unité de commande est réalisée de sorte qu'elle déclenche l'ouverture de la pince (40), après que la membrane (13) de la chambre de mesure (12) et le capteur de pression (14) soient réunis par les troisièmes moyens.

24. Dispositif selon une quelconque des revendications 17 à 23, **caractérisé en ce que** l'unité de commande est réalisée de sorte que, avant la fermeture de la pince (40) et après l'ouverture de la pince (40), elle déclenche respectivement une mesure de pression dans la chambre de mesure (12) et **en ce qu'**un dispositif est prévu pour l'émission d'un message d'erreur, qui est réalisé de sorte qu'il est activé par l'unité de commande lorsque les deux valeurs de pression ne coïncident pas ou ne se trouvent pas dans une zone prescrite.

25. Dispositif selon une quelconque des revendications 14 à 24, **caractérisé en ce que** l'unité de commande est réalisée de sorte qu'elle soumet le capteur de pression (14) à un contrôle de fonctionnement après l'activation des seconds moyens et avant l'activation des troisièmes moyens.

26. Dispositif selon une quelconque des revendications 14 à 25, **caractérisé en ce que** la chambre de mesure (12) est réalisée comme élément jetable ou fait partie d'un élément jetable.

27. Dispositif selon une quelconque des revendications 14 à 26, **caractérisé en ce que** le capteur de pression (14) est réalisé comme élément réutilisable ou fait partie d'un élément réutilisable.

28. Appareil, notamment appareil médical avec au moins un dispositif selon une quelconque des revendications 14 à 27.

29. Appareil selon la revendication 28, **caractérisé en ce qu'**il s'agit d'un appareil de dialyse, d'un appareil d'assistance hépatique ou d'un appareil destiné au traitement de l'asphérèse.

30. Utilisation d'une chambre de mesure (12) limitée par une membrane flexible (13) avec une arrivée de fluide et une évacuation de fluide en tant que composants d'un dispositif manométrique (10) dans un procédé selon une quelconque des revendications 1 à 13 et/ou dans un dispositif selon une quelconque des revendications 14 à 27 et/ou dans un appareil selon une quelconque des revendications 28 ou 29

31. Utilisation selon la revendication 30, **caractérisé en ce que** la chambre de mesure (12) est réalisée comme élément jetable et rejetée après la fin d'un traitement de préférence.
